# EUROPÄISCHE PATENTSCHRIFT

(11) **EP 1 748 725 B1**
(45) Veröffentlichungstag und Bekanntmachung des Hinweises auf die Patenterteilung: **28.11.2018**
(21) Anmeldenummer: 05743233.8
(22) Anmeldetag: 27.04.2005
(51) Int. Cl.: A61B 5/00

(54) **VORRICHTUNG ZUR ERFASSUNG DER SCHWERE EINER ERKRANKUNG**
DEVICE FOR DETECTING THE GRAVITY OF AN ILLNESS
DISPOSITIF POUR DETERMINER LA GRAVITE D'UNE MALADIE

(30) Priorität: 22.05.2004 DE 102004025200
(43) Veröffentlichungstag der Anmeldung: 07.02.2007
(73) Patentinhaber: Löwenstein Medical Technology S.A., 2557 Luxembourg (LU)
(72) Erfinder: SCHWAIBOLD, Matthias, 76137 Karlsruhe (DE); SCHÖLLER, Bernhard, 76135 Karlsruhe (DE); GROTE, Ludger, S-41268 Göteborg (SE)
(74) Vertreter: Klickow & Wetzel PartGmbB
(86) Internationale Anmeldenummer: PCT/DE2005/000768
(87) Internationale Veröffentlichungsnummer: WO 2005/112739

(56) Entgegenhaltungen:
- WO-A-02/065901
- WO-A-2005/030048
- US-A- 5 842 997
- US-A1- 2002 095 094
- US-A1- 2002 173 707
- US-B1- 6 363 270

## Beschreibung

Die Erfindung betrifft eine Vorrichtung zur Erfassung der Schwere von Schlafatemstörungen.

Bei einer Vielzahl von Erkrankungen, beispielsweise auch bei Erkrankungen im Zusammenhang mit dem Schlaf eines Patienten, bestehen Abhängigkeiten zu kardiovaskulären Erkrankungen. Darüber hinaus bestehen auch diverse Wechselwirkungen mit Beeinträchtigungen der Lebensqualität des Patienten, wobei sich Ursachen und Wirkungen jeweils wechselseitig bedingen.

Das Ausmaß sowie die Intensität derartiger Wechselwirkungen lassen sich bislang lediglich statistisch für eine Vielzahl von Patienten, jedoch nicht für einen bestimmten individuellen Patienten erfassen.

Es sind bereits verschiedene Meßverfahren bekannt, um einzelne Parameter zu erfassen, die sich auf die autonome Regulation des kardiovaskulären Systems beziehen. Derartige Meßverfahren werden beispielsweise in der US 5,862,805, der WO 91/11956, der US 2002-0029000, der WO 02/067776 sowie der EP 0 995 592 beschrieben. Es sind bislang jedoch keine Verfahren und keine Vorrichtungen bekannt geworden, die eine umfassende Auswertung der einzelnen Meßwerte zur umfassenden Berücksichtigung der Einzelfaktoren bei der Ermittlung einer Schwere der Erkrankung betreffen.

Insbesondere existieren keinerlei Vorrichtungen, die aus der Kombination der von ihnen gemessenen Parameter Vorhersagen über das individuelle Risiko ermöglichen, aufgrund der vorliegenden Erkrankung, welche möglicher- aber nicht notwendigerweise mit derselben Vorrichtung diagnostiziert wird, Folgeerkrankungen zu entwickeln, welche die Lebensqualität bzw. Lebenserwartung beeinträchtigen.

Ebenso existieren keinerlei Vorrichtungen, die aus der Kombination der von ihnen gemessenen Parameter Aussagen darüber ermöglichen, aus den für die Erkrankung, welche möglicher- aber nicht notwendigerweise mit derselben Vorrichtung diagnostiziert wird, existierenden Therapieverfahren und Therapiedosierungen die individuell optimale auszuwählen.

Ebenso existieren keinerlei Vorrichtungen, die aus der Kombination der von ihnen gemessenen Parameter Aussagen über den Therapieerfolg bei einer Erkrankung, welche möglicher- aber nicht notwendigerweise mit derselben Vorrichtung diagnostiziert wird, ermöglichen.

Aus der WO 02/065901 sind bereits eine Vorrichtung und ein Verfahren zur Erfassung und Analyse von physiologischen Signalen bekannt. Basierend auf den ermittelten Daten wird das Risiko einer Erkrankung an Schlafapnoe ermittelt. Zusätzlich zur Auswertung der meßtechnisch ermittelten Parameter wird vom Patienten ein Fragebogen ausgefüllt und in die Auswertung einbezogen.

Aus der US 2002/173,707 ist ein Analyseverfahren zur Ermittlung des Schweregrades einer Schlafapnoe bekannt. Durch Anwendung der Pulsoximetrie werden hierbei Blutsättigungswerte bestimmt.

Aus der WO 02/065901 A sowie der US 2002/173707 A1 sind jeweils Vorrichtungen zur Erfassung von Schlafatemstörungen unter Verwendung eines Fingersensors bekannt.

Aufgabe der vorliegenden Erfindung ist es daher, eine Vorrichtung der einleitend genannten Art derart zu konstruieren, dass eine individuelle Anwendbarkeit für einen Patienten ermöglicht wird.

Diese Aufgabe wird erfindungsgemäß durch die Merkmalskombination von Patentanspruch 1 gelöst.

Im Gegensatz zur Diagnose einer Erkrankung bedeutet der Begriff "Schwere" erfindungsgemäß nicht nur die reine Häufigkeit und Stärke des Auftretens von Schlafatemstörungen. Vielmehr wird anhand der erfaßten autonomen Regulation des kardiovaskulären Systems erkannt, wie hoch das individuelle Risiko der untersuchten Person ist, Folgeerkrankungen zu erleiden, welche die Lebensqualität bzw. Lebenserwartung beeinträchtigen. Ebenso kann anhand der ermittelten Schwere der Schlafatemstörung abgeleitet werden, welche Therapieform und Therapiedosierung für die Person optimal ist. Außerdem kann der Erfolg einer bereits bestehenden Therapie gemessen werden.

Eine Signalauswertung über eine vorgebbare Zeitspanne kann dadurch unterstützt werden, dass der Sensor mit einer ersten Speichereinheit zur Abspeicherung eines erfassten Meßsignals verbunden ist.

Eine Signalverarbeitung kann ebenfalls dadurch unterstützt werden, dass die Kalkulationseinheit mit einer zweiten Speichereinheit zur Abspeicherung des ermittelten Zustandsparameters verbunden ist.

Zur Durchführung einer Verlaufsanalyse wird vorgeschlagen, dass der Indexermittler einen Verlaufsanalysator zur Analyse des zeitlichen Verlaufes des Zustandsparameters aufweist.

Zur weiteren Verbesserung der Auswertungsmöglichkeiten wird vorgeschlagen, dass der Indexermittler mit einer dritten Speichereinheit zur Abspeicherung des errechneten Index versehen ist.

Ein typischer Auswertungsablauf erfolgt dadurch, dass der Amplitudenverlauf des Meßsignals ausgewertet wird.

Ebenfalls ist daran gedacht, dass eine Steigung des Meßsignals ausgewertet wird.

Gemäß einer weiteren Ausführungsvariante ist vorgesehen, dass eine Frequenz des Meßsignals ausgewertet wird.

Hinsichtlich der Aussageempfindlichkeit erweist es sich insbesondere als vorteilhaft, dass eine Änderungsintensität des Meßsignals ausgewertet wird.

Eine umfassende Signalauswertung kann dadurch erfolgen, dass eine Mustererkennung durchgeführt wird.

Eine Möglichkeit zur Signalerfassung besteht darin, dass ein EEG-Signal ausgewertet wird.

Ebenfalls ist daran gedacht, dass ein EKG-Signal ausgewertet wird.

Zur Erfassung von respiratorischen Parametern ist vorgesehen, dass ein Atmungsverlauf ausgewertet wird.

Ein einfach zu realisierendes Meßprinzip besteht darin, dass eine optische Dichte mindestens eines Körperbereiches ausgewertet wird.

Gemäß einem typischen Auswertungsverfahren ist vorgesehen, dass eine Signalauswertung im Hinblick auf vorliegende periodische Signalanteile durchgeführt wird.

Eine Auswertung besonders aussagefähiger Signalverläufe erfolgt dadurch, dass eine Analyse hinsichtlich einer maximalen Signaländerung durchgeführt wird.

Eine besonders hohe Aussagequalität wird dadurch erreicht, dass von der Auswertungseinheit ein autonomes Ruheprofil der Regulation des kardiovaskulären Systems ermittelt wird. Das autonome Ruheprofil steht dabei im Zusammenhang mit mindestens einem der folgenden Parameter: Schlaftiefe, Schlaffragmentierung, Aktivität des Parasympathikus absolut oder im Verhältnis zum Sympathikus, Blutdruck, Gefäßcompliance.

Insbesondere erweist es sich als vorteilhaft, dass der Index einer kumulativen autonomen Ruheintensität der Regulation des kardiovaskulären Systems zugeordnet ist.

Im Hinblick auf die Erfassung von Atmungsaktivitäten wird vorgeschlagen, dass bei der Ermittlung des autonomen Ruheprofils eine Atmungsaktivität berücksichtigt wird, bzw. die Amplitude der atmungskorrelierten Oszillationen in dem erfaßten Parameter ausgewertet wird.

Die Erfassung besonders aussagekräftiger Ereignisse wird dadurch erreicht, dass eine Signalanalyse hinsichtlich eines Zeitraumes einer Aktivierung des autonomen Systems durchgeführt wird.

Eine nochmalige Steigerung der Aussagequalität kann dadurch erreicht werden, dass bei der Auswertung von Zeiträumen der Aktivierung des autonomen Systems mindestens ein weiterer Atmungsparameter ausgewertet wird. Die Steigerung der Aussagequalität kann dadurch erreicht werden, dass die Änderung verschiedener erfaßter oder aus den erfaßten Parametern abgeleiteter Parameter zum Zeitpunkt einer Aktivierung des autonomen Systems in Stärke, Typ und zeitlicher Abfolge verglichen wird.

Zur Eliminierung von Störungen oder singulären Ereignissen wird vorgeschlagen, dass bei der Ermittlung des Index die kumulative Anzahl und die Intensität der Aktivierungszeiträume des autonomen Systems berücksichtigt wird.

Ein einfacher Meßaufbau wird dadurch unterstützt, dass vom Sensor die Herzfrequenz erfaßt wird.

Insbesondere ist daran gedacht, dass vom Sensor die Variabilität der Herzfrequenz erfaßt wird.

Darüber hinaus ist es auch möglich, dass vom Sensor die Pulstransitzeit erfaßt wird.

Alternativ ist es möglich, dass vom Sensor die Pulsamplitude erfaßt wird.

Zur Störungsunterdrückung trägt es bei, dass ein über einen vorgebbaren Zeitraum erfaßter Mittelwert des vom Sensor erfaßten Meßsignals ausgewertet wird. Die Störunterdrückung kann dadurch weiter verbessert werden, dass vor der Auswertung der erfaßten Parameter eine Artefakterkennung bzw. -beseitigung durchgeführt wird.

Eine Erkennung von kurzfristigen Signaländerungen wird dadurch unterstützt, dass ein innerhalb eines vorgebbaren Zeitintervalls auftretender Maximalwert des vom Sensor erfaßten Meßsignals ausgewertet wird.

Ein einfach anwendbares Meßverfahren besteht darin, dass die Signalerfassung unter Anwendung der Photopletysmographie durchgeführt wird.

Eine Steigerung der Systemempfindlichkeit kann dadurch erreicht werden, dass die Signalauswertung innerhalb mindestens eines vorgebbaren Frequenzbandes durchgeführt wird.

Zur Berücksichtigung von Wechselwirkungen wird vorgesehen, dass von der Auswertungseinheit mindestens ein weiterer Körperparameter ausgewertet wird.

Es ist möglich, dass als weiterer Körperparameter das Geschlecht des Lebewesens ausgewertet wird.

Alternativ oder ergänzend ist es auch möglich, dass als weiterer Körperparameter ein oder mehrere bereits bekannte Risikofaktoren für eine kardiovaskuläre Erkrankung ausgewertet werden.

Alternativ oder ergänzend ist es auch möglich, dass als weiterer Körperparameter ein oder mehrere bereits bekannte Faktoren, die die autonome Regulation beeinflussen, ausgewertet werden, insbesondere die Medikation.

Alternativ oder ergänzend ist es auch möglich, dass als weiterer Körperparameter ein oder mehrere zusätzlich erfaßte Parameter ausgewertet werden, z. B. die arterielle Sauerstoffsättigung.

Eine Berücksichtigung von zeitlich zurückliegenden Ereignissen kann dadurch erfolgen, dass als weiterer Körperparameter die Krankenhistorie des Lebewesens ausgewertet wird.

Eine weitere Steigerung der Aussagequalität kann dadurch erreicht werden, dass als weiterer Körperparameter die Medikation des Lebewesens ausgewertet wird.

In den Zeichnungen sind Ausführungsbeispiele der Erfindung schematisch dargestellt. Es zeigen:
Fig. 1 ein Blockschaltbild zur Veranschaulichung eines typischen Geräteaufbaues und
Fig. 2 ein Ablaufdiagramm zur Veranschaulichung der Durchführung typischer Prozeßschritte bei der Meßwertverarbeitung.

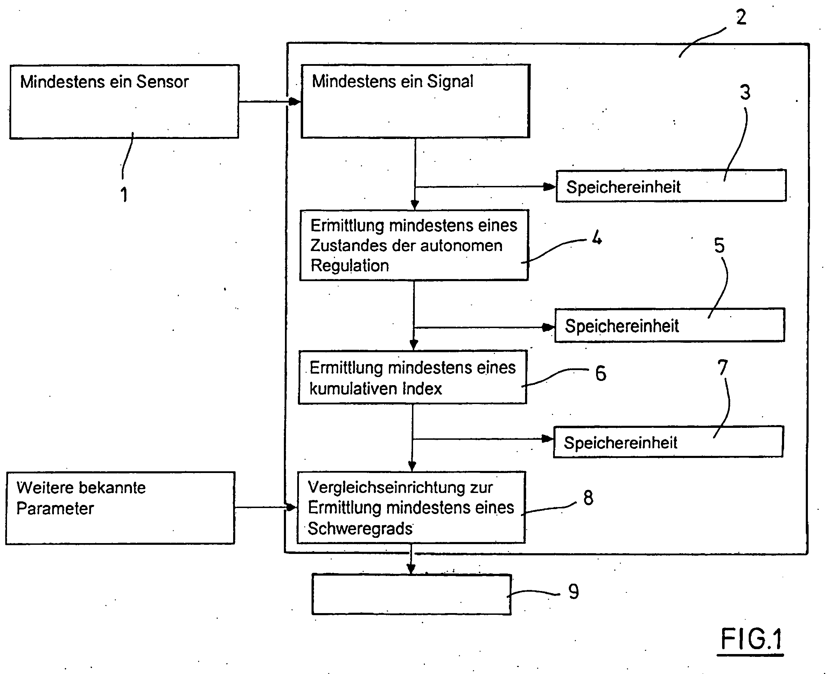

Fig. 1 zeigt ein Ausführungsbeispiel für den strukturellen Aufbau der Vorrichtung zur Erfassung der Schwere von Schlafatemstörungen bei der lediglich ein Sensor verwendet wird, der an eine Auswertungseinrichtung (2) angeschlossen ist.

Das vom Sensor (1) erfaßte Signal wird zunächst im Bereich einer ersten Speichereinheit (3) wenigstens für die Zeitdauer einer nachfolgenden Signalverarbeitung abgespeichert. Die Auswertungseinheit (2) ist mit einer Kalkulationseinheit (4) verbunden, die das vom Sensor (1) erfaßte Signal in einen Zustandsparameter der autonomen Regulation des kardiovaskulären Systems umrechnet. Der Zustandsparameter wird im Bereich einer zweiten Speichereinheit (5) abgespeichert.

Die Kalkulationseinheit (4) ist mit einem Indexermittler (6) verbunden, der eine kumulative Systemeigenschaft der autonomen Regulation des kardiovaskulären Systems ermittelt. Der ermittelte Index wird im Bereich einer dritten Speichereinheit (7) abgespeichert. Von einer Vergleichseinrichtung (8) wird der ermittelte Index unter Berücksichtigung mindestens eines weiteren Parameters des zu untersuchenden Lebewesens analysiert und zu einer Ausgabeeinrichtung (9) übermittelt.

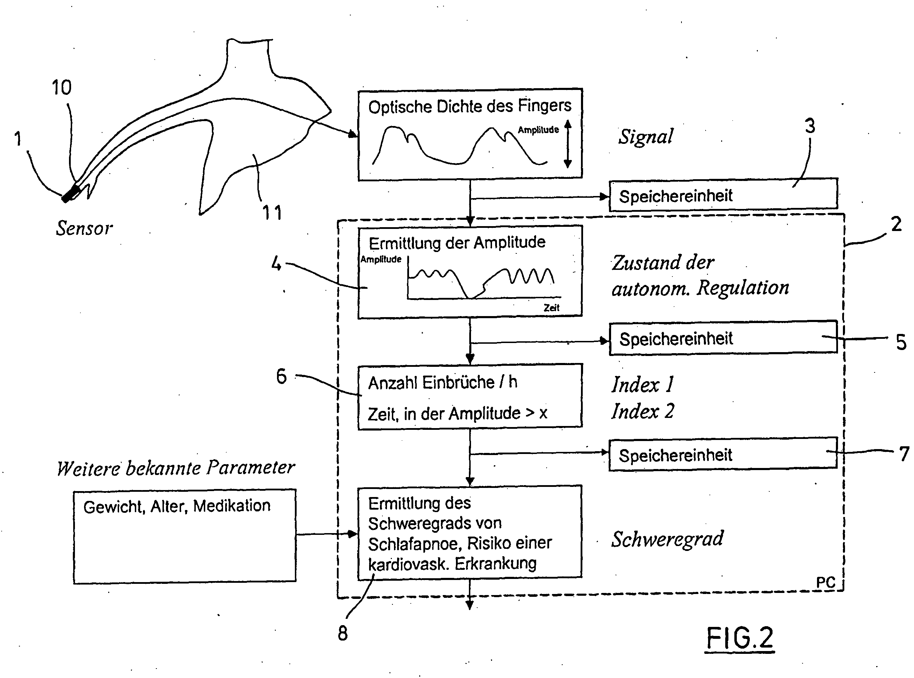

Fig. 2 zeigt ein Ausführungsbeispiel, bei dem im Bereich eines Fingers (10) eines Patienten (11) einen Sensor (1) zur optischen Erfassung der Dichte des Fingers (10) angeordnet ist. Es wird hierbei die Amplitude des Dichteverlaufes in Abhängigkeit von der Zeit erfaßt. Die optische Dichte des Fingers (10) schwankt typischerweise in Abhängigkeit von der durch den Pulsschlag des Patienten (11) verursachten Blutzirkulation. Außerdem schwanken die Stärke der Pulsation sowie die mittlere optische Dichte mit der Atmung des Patienten und bei Aktivierung von Sympathikus und Parasympathikus.

Bei diesem Ausführungsbeispiel ist die erste Speichereinheit (3) außerhalb der Auswertungseinheit (2) angeordnet, die zweite Speichereinheit (5) sowie die dritte Speichereinheit (7) sind als Teil der Auswertungseinheit (2) realisiert. Von der Kalkulationseinheit (4) wird der Amplitudenverlauf eines zugeordneten Zustandsparameters der autonomen Regulation des kardiovaskulären Systems bestimmt. Der Indexermittler (6) bestimmt die Anzahl der Amplitudeneinbrüche je Stunde sowie die Zeit, in der die betreffenden Amplitudenwerte oberhalb eines vorgegebenen Grenzwertes liegen. Es werden somit bei diesem Ausführungsbeispiel ein erster Index sowie ein zweiter Index ermittelt.

Von der Vergleichseinrichtung (8) werden unter Berücksichtigung des ersten Index und des zweiten Index sowie unter Berücksichtigung weiterer bekannter Parameter des Patienten (11), nämlich von Gewicht und Alter und eventuell einer Medikation, der Schweregrad einer vorhandenen Schlafatemstörung bzw. ein Erkrankungsrisiko ermittelt. Im vorliegenden Ausführungsbeispiel wird der Schweregrad von Schlafapnoe ermittelt.

Insbesondere die Variabilität der erfaßten Parameter während normaler Schlafphasen und solcher Schlafphasen, die durch respiratorische Ereignisse fragmentiert sind, gibt Aufschluß über bereits vorliegende oder zu erwartende Schädigungen der autonomen Regulation sowie das Risiko für Folgeerkrankungen. Es wird sowohl die Stärke der zyklischen Variabilität, die unter anderem mit der Atmung korreliert ist, als auch der transienten Variabilität, die unter anderem mit Aufwachreaktionen verbunden ist, ausgewertet und mit der individuell aufgrund von Alter, Gewicht, Krankengeschichte etc. erwarteten Variabilität verglichen.

Dadurch kann außerdem der individuelle Therapiebedarf und die am besten geeignete Therapieform und Therapiedosierung ermittelt werden: Medikation, CPAP-Therapie, CPAP-Therapie mit in- und exspiratorisch unterschiedlichem Druckniveau, CPAP-Therapie mit automatischer Druckanpassung mit bzw. ohne Obstruktionserkennung.

Dabei ist es nicht notwendig, dass die Erkrankung mit derselben Vorrichtung diagnostiziert wird. Dies kann z. B. mit einer herkömmlichen Messung von Atmung und Atemgasanstrengung geschehen. Unter Hinzunahme des Parameters Sauerstoffsättigung kann jedoch auch durch eine spezielle Ausführung derselben Vorrichtung ein AHI mit Unterscheidung von zentralen und obstruktiven Ereignissen bestimmt werden. Anhand der Sättigung werden dabei Phasen mit insuffizienter Atmung registriert. Diese können bei unklarem Verlauf durch einen sympathikus-bedingten Einbruch der Blutzirkulation oder Anstieg der Herzfrequenz im Verlauf bzw. am Ende des Ereignisses abgesichert werden. Steigt während des Ereignisses die Amplitude der atmungsbedingten Schwankungen des gemessenen Parameters, so handelt es sich um ein obstruktives Ereignis. Fällt sie ab, so handelt es sich um ein zentrales Ereignis.

Unter Verwendung der erläuterten Vorrichtung sowie des Verfahrens zur Gerätesteuerung ist es möglich, eine automatisierte Beurteilung der Schwere von unterschiedlichen Erkrankungen durchzuführen. Beispielsweise sind dies Schlafatemstörungen wie obstruktive Schlafapnoe, zentrale Schlaf-apnoe, periodische Atmung, Cheyne-Stokes-Atmung, Schnarchen oder Flußlimitierung. Darüber hinaus ist auch eine Anwendung hinsichtlich von kardiovaskulären Erkrankungen möglich, dies sind beispielsweise Hypertension, chronische Herzinsuffizienz, koronare Herzkrankheit, Schlaganfall, Herzinfarkt, Arrhythmie, Kardiomyopatie oder plötzlicher Herztod. Ebenfalls ist eine Anwendung hinsichtlich allgemeiner Atemwegserkrankungen wie COPD oder Asthma möglich. Ein weiteres Anwendungsgebiet ist die Neuropathie.

Entsprechend der jeweiligen Geräteeinstellung ist es möglich, eine Analyse entweder hinsichtlich des Vorliegens oder des nicht Vorliegens einer bestimmten Erkrankung durchzuführen. Ebenfalls ist es möglich, das Risiko einer weiteren Erkrankung in Abhängigkeit vom Schweregrad einer ersten Erkrankung zu ermitteln.

## Patentansprüche

1. Vorrichtung zur Erfassung der Schwere von Schlafatemstörungen, bei der lediglich ein Sensor (1) zur nicht invasiven Messung mindestens eines von der autonomen Regulation des kardiovaskulären Systems eines Lebewesens abhängigen Signals verwendet ist, wobei der Sensor (1) an eine Auswertungseinheit (2) angeschlossen ist, wobei die Auswertungseinheit (2) eine Kalkulationseinheit (4) zur Umrechnung des Signals in einen Zustandsparameter aufweist und die Auswertungseinheit (2) einen Indexermittler (6) zur Ermittlung mindestens einer kumulativen Systemeigenschaft aufweist und eine Vergleichseinrichtung (8) zur Analyse des Index unter Berücksichtigung weiterer Parameter des Lebewesens an eine Ausgabeeinrichtung (9) für eine ermittelte Schwere der Schlafatemstörung angeschlossen ist, wobei der Sensor (1) zur Anwendung im Bereich eines Fingers (10) eines Patienten (11) anordbar ist und zur optischen Erfassung der Dichte des Fingers (10) verwendet ist und wobei die Vorrichtung geeignet ist, die Amplitude des Dichteverlaufes in Abhängigkeit von der Zeit zu erfassen und wobei die Kalkulationseinheit (4) geeignet ist, den Amplitudenverlauf einer Sauerstoffsättigung des Blutes zu bestimmen und wobei der Indexermittler (6) geeignet ist, die Anzahl der Amplitudeneinbrüche je Stunde sowie die Zeit, in der die betreffenden Amplitudenwerte oberhalb eines vorgegebenen Grenzwertes liegen, zu bestimmen, sodass ein erster Index sowie ein zweiter Index ermittelt werden, wobei die Vergleichseinrichtung (8) geeignet ist, unter Berücksichtigung des ersten Index und des zweiten Index sowie unter Berücksichtigung weiterer bekannter Parameter des Patienten (11), nämlich Gewicht und Alter, den Schweregrad der Schlafatemstörung zu ermitteln.

## Claims

1. Apparatus for detecting the severity of sleep-related respiratory disorders, in which use is made of only one sensor (1) for non-invasive measurement of at least one signal that depends on the autonomous regulation of the cardiovascular system of a living being, wherein the sensor (1) is connected to an evaluation unit (2), wherein the evaluation unit (2) comprises a calculation unit (4) for converting the signal into a state parameter and the evaluation unit (2) comprises an index establishing means (6) for establishing at least one cumulative system property and a comparison device (8) for analysing the index taking account of further parameters of the living being is connected to an output device (9) for an established severity of the sleep-related respiratory disorder, wherein the sensor (1) is arrangeable for application in the region of a finger (10) of a patient (11) and is used to optically capture the density of the finger (10) and wherein the apparatus is suitable for capturing the amplitude of the density profile as a function of time and wherein the calculation unit (4) is suitable for determining the amplitude curve of an oxygen saturation of the blood and wherein the index establishing means (6) is suitable for determining the number of amplitude drops per hour and the time during which the relevant amplitude values lie above a predetermined threshold such that a first index and a second index are established, wherein the comparison device (8) is suitable for establishing the degree of severity of the sleep-related respiratory disorder taking account of the first index and the second index and taking account of further known parameters of the patient (11), specifically weight and age.

## Revendications

1. Arrangement pour détecter la gravité de dysfonctionnements respiratoires du sommeil, avec lequel seulement un capteur (1) est utilisé pour la mesure non invasive d'au moins un signal dépendant de la régulation autonome du système cardiovasculaire d'un être vivant, le capteur (1) étant raccordé à une unité d'interprétation (2), l'unité d'interprétation (2) possédant une unité de calcul (4) destinée à convertir le signal en un paramètre d'état et l'unité d'interprétation (2) possédant un déterminateur d'index (6) destiné à déterminer au moins une propriété cumulative du système et un dispositif de comparaison (8) destiné à analyser l'index en tenant compte d'autres paramètres de l'être vivant étant raccordé à un dispositif de sortie (9) pour une gravité déterminée du dysfonctionnement respiratoire du sommeil, le capteur (1) pouvant être disposé dans la zone d'un doigt (10) d'un patient (11) pour son utilisation et étant utilisé pour l'acquisition optique de la densité du doigt (10) et l'arrangement étant conçu pour détecter l'amplitude de la courbe de densité en fonction du temps et l'unité de calcul (4) étant conçue pour déterminer la courbe d'amplitude d'une saturation en oxygène du sang et le déterminateur d'index (6) étant conçu pour déterminer le nombre de chutes de l'amplitude par heure ainsi que la durée pendant laquelle les valeurs d'amplitude concernées se trouvent au-dessus d'une valeur limite prédéfinie, de sorte qu'un premier index ainsi qu'un deuxième index soient déterminés, le dispositif de comparaison (8) étant conçu pour déterminer le degré de gravité du dysfonctionnement respiratoire du sommeil en tenant compte du premier index ainsi que du deuxième index et aussi en tenant compte d'autres paramètres connus du patient (11), à savoir le poids et l'âge.
